# EUROPÄISCHE PATENTANMELDUNG

(11) **EP 3 613 449 A1**
(43) Veröffentlichungstag der Anmeldung: **26.02.2020**
(21) Anmeldenummer: 18190387.3
(22) Anmeldetag: 23.08.2018
(51) Int. Cl.: A61L 31/02, A61L 31/08, A61L 31/10, A61L 31/16

(54) **VERBESSERUNG DER POLYMERSCHICHT BEI DEGRADIERBAREN VORRICHTUNGEN**

(71) Anmelder: BIOTRONIK AG, 8180 Bülach (CH)
(72) Erfinder: Göpfert, André, 18292 Alt Sammit (DE)
(74) Vertreter: Randoll, Sören

(57) **Zusammenfassung**

Die Erfindung betrifft ein Implantat, insbesondere ein endovaskulares Implantat, mit einem abbaubaren Grundkörper; einer ersten abbaubaren Beschichtung umfassend mindestens ein abbaubares Polymer; und einem antiproliferativen Wirkstoff, wobei die erste abbaubare Beschichtung den abbaubaren Grundkörper im Wesentlichen vollständig umschließt, und der antiproliferative Wirkstoff auf der abluminalen Seite des Grundkörpers auf die erste abbaubare Beschichtung aufgetragen ist. Die Erfindung betrifft weiterhin ein Verfahren zur Herstellung eines solchen Implantats.

## Beschreibung

Die Erfindung betrifft ein Implantat, insbesondere ein endovaskuläres Implantat, und ein Verfahren zu dessen Herstellung.

Medizinische Implantate oder Endoprothesen, insbesondere Stents, sind in großer Vielfalt aus dem Stand der Technik bekannt.

Stents werden vor allem bei Herz-Kreislauferkrankungen verwendet, in welchen Blutgefäße oder Hohlorgane offengehalten werden sollen. Je nach beabsichtigter Einsatzdauer können hierbei permanente Stents verwendetet werden, die im Körper verbleiben oder operativ entfernt werden müssen, oder abbaubare Stents, welche nach einer gewissen Zeit vom Körper abgebaut werden.

Vor allem bei Blutgefäß-Stents besteht jedoch das Risiko eines Verschlusses. Dies kann beispielsweise durch die Bildung eines Thrombus geschehen, da der Stent als Fremdkörper die Blutgerinnung anregen kann. Weiterhin kann es im Laufe der Zeit zu einem allmählichen Zuwachsen und Verschluss durch die Neubildung von Bindegewebe kommen.

Um die Neubildung des Bindegewebes zu verhindern werden gegenwärtig Drug-Eluting-Stents, verwendetet, die mit aktiven Substanzen wie Antiproliferativa, Zytostatika, Immunmodulatoren und ähnlichen beschichtet sind, welche die vorgenannten Substanzen an die Umgebung freigeben.

Permanente Stents aus beispielsweise Metallen oder Metalllegierungen können weiterhin Thrombosen verursachen.

Ein Vorteil hierbei bieten die oben genannten abbaubaren oder bioresorbierbaren Stents, welche nur so lange im Gefäß verbleiben, wie es klinisch notwendig ist. Die Haltbarkeit bzw. Abbaugeschwindigkeit solcher abbaubarer Stents ist wesentlich von den gewählten Materialien abhängig. Weiterhin müssen die verwendeten Materialien vom Körper gut vertragen werden und müssen eine bestimmte Verformbarkeit und Festigkeit aufweisen. Bevorzugte Materialien sind insbesondere biokompatible Metalle wie Magnesium, die eine Rolle im Stoffwechsel spielen. Reines Magnesium oder Magnesiumlegierungen weisen jedoch meist eine hohe Abbaugeschwindigkeit auf.

Daher sind insbesondere Magnesiumstents vorzugsweise mit einem abbaubaren Polymer beschichtet, um die Abbaugeschwindigkeit zu verringern bzw. genauer steuern zu können.

Basierend auf diesem Hintergrund ist die Aufgabe der vorliegenden Erfindung, ein Implantat zur Verfügung zu stellen, welches rückstandslos abbaubar ist und ein geringeres Risiko zur Ausbildung einer Restenose aufweist.

Diese Aufgabe wird durch ein hierin vorgeschlagenes Implantat sowie durch ein hierin vorgeschlagenes Verfahren gelöst.

Demgemäß wird ein Implantat vorgeschlagen, das folgendes umfasst:
- einen abbaubaren Grundkörper;
- eine erste abbaubare Beschichtung umfassend mindestens ein abbaubares Polymer; und
- einen antiproliferativen Wirkstoff.

Erfindungsgemäß ist dabei vorgesehen, dass die erste abbaubare Beschichtung den abbaubaren Grundkörper im Wesentlichen vollständig umschließt, und der antiproliferative Wirkstoff auf der abluminalen Seite des Grundkörpers auf die erste abbaubare Beschichtung aufgetragen ist.

Durch eine solche Anordnung der Beschichtung ist es möglich die Regime der Steuerung der Degradation und der Wirkstoffabgabe zu entkoppeln. Bekannte Stentbeschichtungen sehen vor, dass der Wirkstoff in der Polymermatrix gemischt vorliegt. In einer solchen Anordnung wird die Wirkstoffabgabe maßgeblich durch die Degradation der polymeren Beschichtung bestimmt. Des Weiteren wird die Degradationssteuerung des abbaubaren Gerüstes der Gefäßstütze durch die polymere Beschichtung durch die spezifischen Eigenschaften des Wirkstoffes in der Beschichtung ebenfalls beeinflusst. Durch die getrennte Auftragung der polymeren Beschichtung und des Wirkstoffes auf der abluminalen Seite wird zum einen erreicht, dass man die Degradation des Gerüstes der Gefäßstütze durch die polymere Beschichtung exakt steuern kann, da die Beschichtung ohne gelösten Wirkstoff vorliegt. Zum anderen kann die Wirkstoffabgabe nur in das Gewebe auf der abluminalen Seite unabhängig von den Degradationseigenschaften der polymeren Beschichtung erfolgen.

Der Begriff "im Wesentlichen vollständig" im Sinne der vorliegenden Erfindung bedeutet insbesondere, dass der Grundkörper zu mindestens 80 %, 85 %, 90 %, 95 %, 98 % oder 99 % von der ersten abbaubaren Beschichtung umschlossen bzw. bedeckt ist.

Der Begriff "abbaubar" im Sinne der Erfindung bedeutet insbesondere, dass die betreffenden Komponenten durch chemische Prozesse im lebenden Organismus, vor allem durch die mit den Komponenten in Kontakt gelangenden Körperflüssigkeiten, allmählich aufgelöst werden, und die Abbauprodukte der aufgelösten Komponenten vom lebenden Organismus resorbiert oder ausgeschieden werden.

Der Grundköper kann hierbei jede Gestalt bzw. Form aufweisen, welche im Wesentlichen von dem gewünschten Einsatzort und/oder Verwendungszweck im Körper eines Patienten abhängig ist.

Vorzugsweise ist das gesamte Implantat vollständig abbaubar.

In einer Ausführungsform des erfindungsgemäßen Implantats ist vorgesehen, dass der Grundkörper vollständig von der ersten abbaubaren Beschichtung umschlossen ist.

In einer Ausführungsform des erfindungsgemäßen Implantats ist vorgesehen, dass die erste abbaubare Beschichtung Polymilchsäure (CAS Nr. 26100-51-6), insbesondere Poly(L-Milchsäure), Polylactid-co-Glycolid oder Poly-ε-Caprolacton umfasst oder im Wesentlichen daraus besteht.

Eine Beschichtung des erfindungsgemäßen Implantat mit Polymilchsäure oder Poly(Lactid-co-Glykolid) ist besonderes bei besonders hydrophoben antiproliferativen Wirkstoffen vorteilhaft, da hierbei eine abluminale Auftragung des Wirkstoffes ohne vorzeitige Delamination möglich ist.

In einer Ausführungsform des erfindungsgemäßen Implantats ist vorgesehen, dass der antiproliferative Wirkstoff ein mTOR-Inhibitor oder ein Zytostatikum ist. In einer Ausführungsform des erfindungsgemäßen Implantats ist vorgesehen, dass das Zytostatikum Paclitaxel ist.

In einer Ausführungsform des erfindungsgemäßen Implantats ist vorgesehen, dass der mTOR-Inhibitor ausgewählt ist aus:
- Sirolimus (CAS-Nr. 53123-88-9);
- Everolimus (CAS-Nr.159351-69-6);
- Temsirolimus (CAS-Nr. 162635-04-3);
- Ridaforolimus (CAS-Nr.572924-54-0);
- Zotarolimus; oder
- Biolimus A9.

In einer Ausführungsform des erfindungsgemäßen Implantats ist vorgesehen, dass der antiproliferative Wirkstoff bzw. der mTOR-Inibhitor eine Verbindung nach Formel I ist 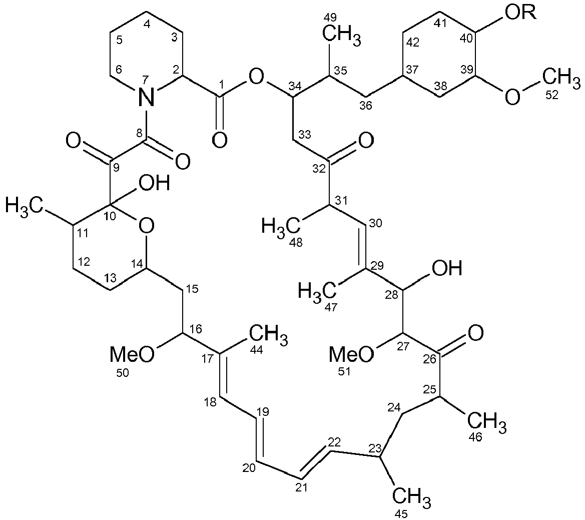 wobei R C(O)-(CH2)n-X ist, n 0, 1 oder 2 ist, X ein zyklischer Kohlenwasserstoff mit 3-9 Kohlenstoffatomen ist, welcher optional eine oder mehrere ungesättigte Bindungen enthält.

In einer Ausführungsform des erfindungsgemäßen Implantats ist vorgesehen, dass die Verbindung nach Formel I die folgende Struktur aufweist: 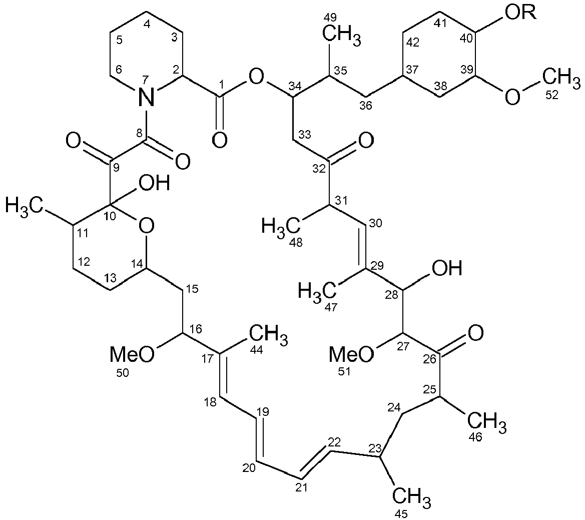 und R mit C(O)-(CH₂)ₙ₋X eine der folgenden Strukturen aufweist: 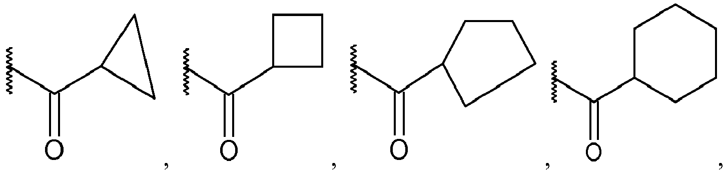 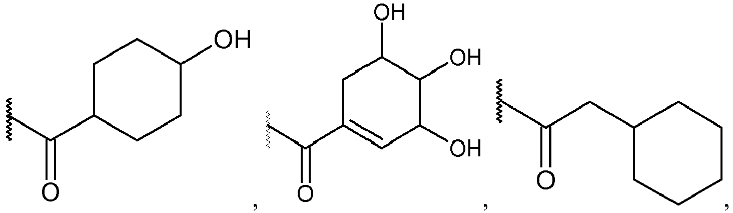 und

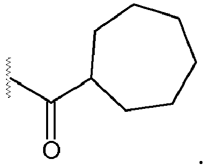

In einer Ausführungsform des erfindungsgemäßen Implantats ist vorgesehen, dass die erste Beschichtung (4) Poly(L-Milchsäure) mit einem geeigneten Molekulargewicht aus dem gesamten verfügbaren Bereich gewählt werden kann, je nach Eigenschaften des Grundkörpers.

In einer bevorzugten Ausführungsform umfasst die erste Beschichtung (4) Poly(L-Milchsäure) mit einer inhärenten Viskosität im Bereich von 0.5 dl/g bis 4.0 dl/g, weiter bevorzugt im Bereich von 0.7 dl/g bis 3.8 dl/g, oder die erste Beschichtung besteht im Wesentlichen aus den zuvor genannten Poly(L-Milchsäuren). In einer Ausführungsform umfasst oder besteht die erste Beschichtung (4) Poly(L-Milchsäure) mit einer inhärenten Viskosität im Bereich von 0.9 dl/g bis 3.8 dl/g. In einer weiteren Ausführungsform umfasst oder besteht die erste Beschichtung (4) Poly(L-Milchsäure) mit einer inhärenten Viskosität im Bereich von 0.7 dl/g bis 1.2 dl/g.

Für das erfindungsgemäße Implantat ist es vorgesehen, dass der Grundkörper aus einem abbaubaren Metall oder einer abbaubaren Metalllegierung hergestellt ist. In einer bevorzugten Ausführungsform ist das Metall oder die Metalllegierung ausgewählt aus der Gruppe umfassend oder bestehend aus Magnesium, Eisen und Zink oder Legierungen daraus, wobei die zuvor genannten Metalle deren größte Bestandteile darstellen. In einer besonders bevorzugten Ausführungsformumfasst das Implantat einen Grundkörper, der Magnesium oder eine abbaubare Magnesiumlegierung umfasst oder im Wesentlichen daraus besteht. Vorteilhafterweise werden beim Abbau des erfindungsgemäßen Implantats Magnesiumionen am Einsatzort freigesetzt, welche dort einen vorteilhaften antithrombischen Effekt hervorrufen können.

In einer Ausführungsform des erfindungsgemäßen Implantats ist vorgesehen, dass der Grundkörper in Form eines hohlzylinderförmigen Gitters ausgebildet ist.

In einer Ausführungsform des erfindungsgemäßen Implantats ist vorgesehen, dass das Implantat ein endovaskulares Implantat ist. In einer Ausführungsform des erfindungsgemäßen Implantats ist vorgesehen, dass das Implantat ein Stent ist.

In einer Ausführungsform wird ein Implantat und vorzugsweise ein Stent vorgeschlagen, umfassend
- einen abbaubaren Grundkörper aus Magnesium oder einer Magnesiumlegierung;
- eine erste abbaubare Beschichtung umfassend oder bestehend aus Poly(L-Milchsäure) mit einem Molekulargewicht im Bereich von 0.7 dl/g bis 1.2 dl/g; und
- einen antiproliferativen Wirkstoff, vorzugsweise einem Wirkstoff der Formel I wie hierin beschrieben.

In einer weiteren Ausführungsform wird ein Implantat und vorzugsweise ein Stent vorgeschlagen, umfassend
- einen abbaubaren Grundkörper aus Eisen oder einer Eisenlegierung;
- eine erste abbaubare Beschichtung umfassend oder bestehend aus Poly(L-Milchsäure) mit einem Molekulargewicht im Bereich von 0.9 dl/g bis 3.8 dl/g; und
- einen antiproliferativen Wirkstoff, vorzugsweise einem Wirkstoff der Formel I wie hierin beschrieben.

Ein weiterer Aspekt der vorliegenden Erfindung betrifft ein Verfahren zur Herstellung des erfindungsgemäßen Implantats. Das Verfahren umfasst die Schritte:
- Bereitstellen eines abbaubaren Grundkörpers;
- Auftragen einer ersten abbaubaren Beschichtung umfassend mindestens ein abbaubares Polymer im Wesentlichen auf die gesamte Oberfläche des abbaubaren Grundköper; und
- Auftragen eines antiproliferativen Wirkstoffes auf der abluminalen Seite des Grundkörpers auf die erste abbaubare Beschichtung.

In einer Ausführungsform des erfindungsgemäßen Verfahrens ist vorgesehen, dass die erste abbaubare Beschichtung und/oder der antiproliferative Wirkstoff durch Aufsprühen, Zerstäuben, Eintauchen oder Pipettieren aufgetragen wird.

Nachfolgend sollen schließlich weitere Merkmale sowie Vorteile der vorliegenden Erfindung anhand von einzelnen Ausführungsformen bzw. Beispielen der Erfindung erläutert werden.

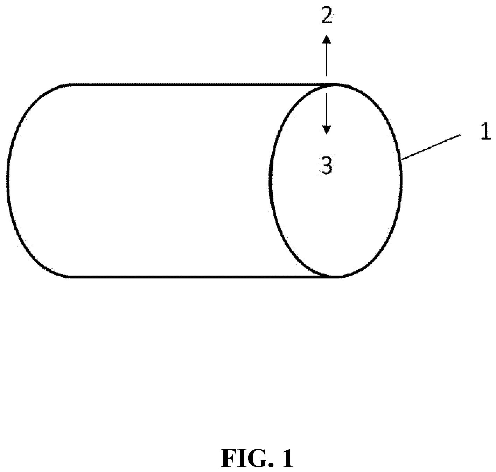

Erstes Beispiel:
Das erfindungsgemäße Implantat ist schematisch in Figur 1 dargestellt. In dieser Ausführungsform der Erfindung ist das Implantat als Gefäßstütze oder Stent ausgebildet, wobei der Grundkörper 1 die Gestalt eines gitterförmigen Hohlzylinders hat, der durch eine Vielzahl von einzelnen Streben 1 gebildet wird. Der Grundköper weist hierbei eine luminale 3, das heißt dem Lumen des Gefäßes, in welchem der Stent eingesetzt werden soll, zugewandte Seite auf sowie eine abluminale 2, das heißt der Gefäßwand zugewandte Seite. Der Grundkörper besteht dabei aus einer abbaubaren Magnesiumlegierung.

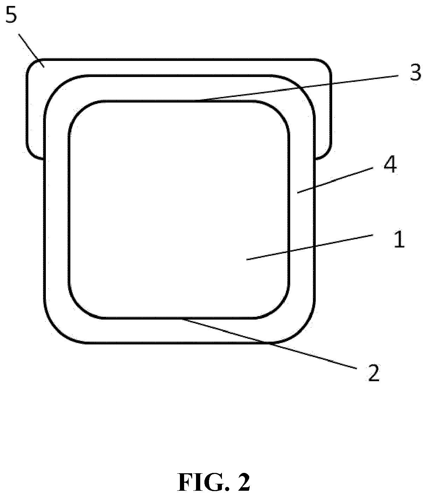

Figur 2 zeigt schematisch eine Strebe 1 des Grundkörpers. Hierbei ist die Strebe 1, wie auch der gesamte Grundkörper, von einer ersten abbaubaren Beschichtung, vorzugsweise aus PLLA oder PLGA, umschlossen. Diese Beschichtung dient vorzugsweise als Passivierungsschicht, welche den Abbau des Implantats vorteilhafterweise verzögern kann, da erst nach Abbau der ersten Beschichtung, der Grundkörper im Kontakt mit den umgebenden Körperflüssigkeiten gelangen kann, welche dann den Abbau des Grundkörpers verursacht.

Erfindungsgemäß ist dabei vorgesehen, dass ein antiproliferativer Wirkstoff 5, vorzugsweise ein Derivat von Rapamycin, auf die abluminale Seite 2 des Grundkörpers 1 auf die erste abbaubare Beschichtung 4 aufgetragen ist. Diese Auftragung auf die abluminale Seite ermöglicht eine gezielte Medikamentierung bzw. gezielt gewebeseitige Abgabe des Wirkstoffes 5, welcher dann genau am Bestimmungsort seine Wirkung entfalten kann. Dadurch kann vorteilhafterweise die Einheilung des Stens effektiv beschleunigt werden. Auch ist es dadurch in vorteilhafter Weise möglich, die Dual-Anti-Platelet-Therpie (DAPT) zu verkürzen.

### Beispiel 2

Besonders vorteilhafte antiproliferative Wirkstoffe werden in der folgenden Tabelle 2 dargestellt.

**Tabelle 2**

| CRC-015 Spezies | | |
|---|---|---|
| Hauptstruktur | R ist C(O)-(CH2)n-X mit einem der folgenden Strukturen | Spezies |
| 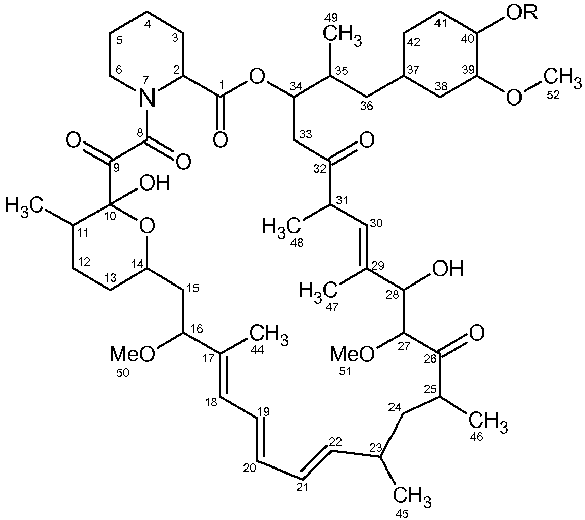 | 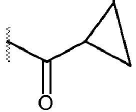 | CRC-015a |
| | 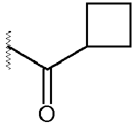 | CRC-0 15b |
| | 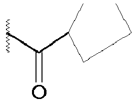 | CRC-015c |
| | 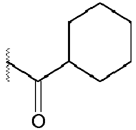 | CRC-015d |
| | 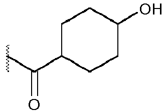 | CRC-015e |
| | 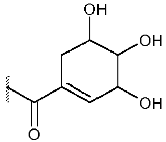 | CRC-015f |
| | 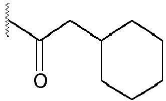 | CRC-015g |
| | 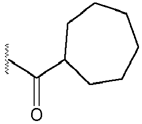 | CRC-01 5h |

Der Begriff CRC-015 soll hier einen Gruppe von Verbindungen beschreiben und bezieht sich auf die einzelnen Spezies oder Verbindung aus Tabelle 2: CRC-015a, CRC-015b, CRC-015c, CRC-015d, CRC-015e, CRC-015f, CRC-015g, und CRC-015h.

Vorzugsweise wird eine CRC_015 Verbindung mit einem wasserlöslichen Lösungsvermittler und einem humanen Serumprotein formuliert. Dafür wird die Verbindung in EtOH gelöst und eine physiologische Kochsalzlösung mit dem humanen Serumprotein gegeben.

## Patentansprüche

1. Implantat mit:
- einem abbaubaren Grundkörper (1);
- einer ersten abbaubaren Beschichtung (4) umfassend mindestens ein abbaubares Polymer; und
- einem antiproliferativen Wirkstoff (5),
**dadurch gekennzeichnet, dass**
die erste abbaubare Beschichtung (4) den abbaubaren Grundkörper (1) im Wesentlichen vollständig umschließt, und
der antiproliferative Wirkstoff (5) auf der abluminalen Seite (2) des Grundkörpers (1) auf die erste abbaubare Beschichtung (5) aufgetragen ist.

2. Implantat nach Anspruch 1, **dadurch gekennzeichnet, dass** die erste abbaubare Beschichtung (4) Polymilchsäure und/oder Poly(Lactid-co-Glycolid) umfasst oder daraus im Wesentlichen besteht, insbesondere Poly(L-Milchsäure), Poly-ε-Caprolacton oder Poly(L-Lactid-co-Glycolid).

3. Implantat nach einem der vorherigen Ansprüche, **dadurch gekennzeichnet, dass** der antiproliferative Wirkstoff (5) ein mTor-Inhibitor oder ein Zytostatikum, insbesondere Paclitaxel, ist.

4. Implantat nach Anspruch 3, **dadurch gekennzeichnet, dass** der mTor-Inhibitor (5) ausgewählt ist aus:
- Sirolimus;
- Everolimus;
- Zotarolimus
- Temsirolimus;
- Biolimus A9
- Ridaforolimus; oder
- Verbindung nach Formel I: 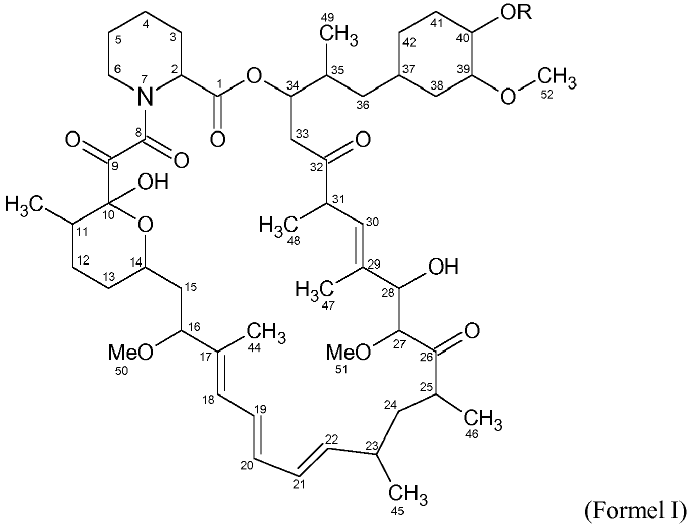 wobei R C(O)-(CH2)n-X ist, n 0, 1 oder 2 ist, X ein zyklischer Kohlenwasserstoff mit 3-9 Kohlenstoffatomen ist, welcher optional eine oder mehrere ungesättigte Bindungen enthält.

5. Implantat nach einem der vorherigen Ansprüche, **dadurch gekennzeichnet, dass** die erste Beschichtung (4) Poly(L-Milchsäure) mit einem mittleren Molekulargewicht im Bereich von X Da bis Y Da umfasst oder im Wesentlichen daraus besteht.

6. Implantat nach einem der vorherigen Ansprüche, **dadurch kennzeichnet, dass** der Grundkörper (1) im Wesentlichen aus einem abbaubaren Metall besteht oder ein abbaubares Metall in Form einer abbaubaren Legierung umfasst.

7. Implantat nach einem der vorherigen Ansprüche, **dadurch kennzeichnet, dass** der Grundkörper (1) Magnesium oder eine abbaubare Magnesiumlegierung umfasst oder im Wesentlichen daraus besteht.

8. Implantat nach einem der vorherigen Ansprüche, **dadurch gekennzeichnet, dass** der Grundkörper (1) in Form eines hohlzylinderförmigen Gitters ausgebildet ist.

9. Implantat nach einem der vorherigen Ansprüche, **dadurch gekennzeichnet, dass** das Implantat ein endovaskulares Implantat ist, insbesondere ein Stent.

10. Verfahren zur Herstellung eines Implantats gemäß einem der Ansprüche 1 bis 9, umfassend die Schritte:
- Bereitstellen eines abbaubaren Grundkörpers
- Auftragen einer ersten abbaubaren Beschichtung umfassend mindestens ein abbaubares Polymer auf mindestens die abluminale Seite des abbaubaren Grundköper,
- Auftragen eines antiproliferativen Wirkstoffes auf die erste abbaubare Beschichtung auf der abluminale Seite des Grundkörpers.

11. Verfahren zur Herstellung eines Implantats nach Anspruch 10, **dadurch gekennzeichnet, dass** die erste abbaubare Beschichtung und /oder der antiproliferative Wirkstoff durch Aufsprühen, Zerstäuben, Eintauchen oder Pipettieren aufgetragen wird.
